# EUROPÄISCHE PATENTSCHRIFT

(11) **EP 2 282 677 B1**
(45) Veröffentlichungstag und Bekanntmachung des Hinweises auf die Patenterteilung: **10.09.2014**
(21) Anmeldenummer: 09761747.6
(22) Anmeldetag: 10.06.2009
(51) Int. Cl.: A61B 10/06, A61B 17/29, A61B 17/00

(54) **MEDIZINISCHES GREIFGERÄT**
MEDICAL GRIPPING DEVICE
APPAREIL MÉDICAL POUR SAISIR

(30) Priorität: 11.06.2008 DE 202008007775 U
(43) Veröffentlichungstag der Anmeldung: 16.02.2011
(73) Patentinhaber: Ovesco Endoscopy AG, 72074 Tübingen (DE)
(72) Erfinder: SCHURR, Marc O., 72072 Tübingen (DE); HO, Chi-Nghia, 70180 Stuttgart (DE); KIRSCHNIAK, Andreas, Dr., 72074 Tübingen (DE); ANHÖCK, Gunnar, 72762 Reutlingen (DE)
(74) Vertreter: Winter, Brandl, Fürniss, Hübner, Röss, Kaiser, Polte - Partnerschaft mbB
(86) Internationale Anmeldenummer: PCT/EP2009/057205
(87) Internationale Veröffentlichungsnummer: WO 2009/150184

(56) Entgegenhaltungen:
- DE-A1- 4 420 284
- US-A- 4 326 530
- US-A- 5 228 451
- US-A- 5 403 332
- US-A- 5 549 636
- US-A- 5 779 648
- US-A- 5 792 165
- US-A1- 2007 260 264
- US-A1- 2007 276 430
- US-B1- 6 673 092

## Beschreibung

### TECHNISCHES GEBIET

Die vorliegende Erfindung bezieht sich auf ein medizinisches Greifgerät und insbesondere auf ein medizinisches Greifgerät mit mehreren Branchen, die einzeln steuerbar sind.

### STAND DER TECHNIK

Heutzutage ist der Einsatz der flexiblen Endoskopie ein gängiger Standard für die diagnostische und therapeutische Behandlung von Erkrankungen des Magen-Darm-Trakts. Dabei wird ein flexibles Endoskop in natürliche Öffnungen des Körpers eingeführt, wie zum Beilspiel Mund und Anus. Da bei dieser Operationsmethode kein direkter Eingriff in das zu operierende Gewebe o.ä. möglich ist, müssen flexible endoskopische Instrumente verwendet werden. Hierbei kommen auch Greifinstrumente zum Einsatz, die beispielsweise Gewebeproben entnehmen können oder womit Gewebe gegriffen und manipuliert werden kann.

Im Stand der Technik gibt es ein Greifgerät, das einen hohlen flexiblen Schaft besitzt, an dessen vorderem Ende zwei Branchen drehbar befestigt sind. Die beiden Branchen bilden eine Art Maul, das sich zu dem vorderen Ende des Greifgeräts öffnen kann. An dem hinteren Ende jeder Branche ist über einen Hebelarm ein zug- und druckfestes Seil montiert. Die beiden Seile sind mit einem Ende eines Bowdenzugs verbunden, der durch den holen Schaft läuft und an seinem anderen Ende mit einem Griff verbunden ist. Mit der Betätigung des Griffs kann das Maul, das sich an dem anderen Ende des Greifgeräts befindet, geöffnet und geschlossen werden. Das ganze Greifgerät wird durch einen Arbeitskanal eines Endoskops in den Körper eingeführt.

Gemäß einem weiteren Instrument aus dem Stand der Technik besitzt ein Greifgerät einen flexiblen Schaft mit einem starren Steg (oder einem starren Maulteil) an einem Ende. An diesem starren Steg ist eine Branche angelenkt, die mittels eines Bowdenzugs bewegbar ist. Der Bowdenzug verläuft in dem flexiblen Schaft und ist an dem anderen Ende des Greifgeräts mit einem Griff verbunden. Mit der Betätigung des Griffs wird die Branche geöffnet und geschlossen. Dieses Greifgerät kann durch einen Arbeitskanal eines Endoskops eingeführt werden.

Die Greifgeräte aus dem Stand der Technik besitzen allerdings einige Nachteile. In der Praxis ist es oft erforderlich, Gewebeteile miteinander zu verbinden oder Öffnungen im Gewebe zu verschließen. Dabei ist es erforderlich, dass die entsprechenden Gewebeteile oder die gegenüberliegenden Randabschnitte der Öffnungen gegriffen und somit fixiert werden können. Ansonsten ist keine kontrollierte Verbindung oder kein kontrollierter Verschluss des Gewebes möglich. Wenn zum Beispiel eine Perforation nicht verschlossen werden kann, muss oftmals ein offener Eingriff vorgenommen werden, um Komplikationen zu vermeiden, die beispielsweise durch eintretende Keime verursacht werden. Da aber die Greifgeräte aus dem Stand der Technik nur jeweils ein Maul besitzen, ist es zwangsläufig erforderlich, dass mindestens zwei dieser Greifgeräte zum Einsatz kommen. Dies ist aber im Hinblick auf den dann erforderlichen (zweiten Zugang) zweiten Arbeitskanal im Endoskop oder ein zusätzliches zweites Endoskop problematisch durch die Enge der natürlichen Körperöffnungen, wie beispielsweise Speiseröhre, Dünn- und Dickdarm. In jüngster Zeit wird an neuen Operationsmethoden, den so genannten NOTES (Natural Orifice Transluminal Endoscopic Surgery) geforscht, bei der versucht wird, ohne einen Hautschnitt in Körperhöhlen wie den Bauchraum zu gelangen, um dort zu operieren. Dabei werden die Zugänge mit Hilfe des flexiblen Instruments durch beispielsweise Magen, Dickdarm oder Vagina geschaffen. Diese Zugänge gilt es nach der Operation effektiv zu verschließen. Das gelingt jedoch nur wenn die entsprechenden Randabschnitte gegriffen und somit fixiert werden können. US-B-6673 092 offenbart ein medizinisches Greifgerät gemäß dem Oberbegriff des Anspruchs 1.

### ZUSAMMENFASSUNG DER ERFINDUNG

Es ist daher eine Aufgabe der Erfindung, ein medizinisches Greifgerät vorzusehen, mit dem mindestens zwei Gewebeteile separat gegriffen und somit fixiert werden können. Eine weitere Aufgabe der Erfindung ist es, einen Handgriff für solch ein medizinisches Greifgerät bereitzustellen, mit dem das medizinische Greifgerät gehalten und betätigt werden kann. Noch eine weitere Aufgabe der vorliegenden Erfindung besteht darin, ein Verbindungselement zwischen medizinischem Greifgerät und Handgriff vorzusehen, mit dem eine einzelne und voneinander unabhängige Betätigung der einzelnen Branchen realisiert werden kann.

Die Aufgabe der vorliegenden Erfindung wird mit einem medizinischen Greifgerät gemäß dem Schutzanspruch 1 gelöst. Weiter vorteilhafte Weiterbildungen des medizinischen Greifgeräts sind Gegenstand der Unteransprüche.

Gemäß einem ersten Aspekt besitzt ein medizinisches Greifgerät einen flexiblen Schaft mit einem vorderen und einem hinteren Ende, einen Steg bestehend aus mindestens einem Stegelement, der an dem vorderen Ende des Schafts befestigt ist, mindestens zwei Branchen, die an dem Steg angelenkt sind, und mindestens zwei flexible Steuermechanismen, die zumindest teilweise in dem Schaft angeordnet sind. Darüber hinaus ist an dem hinteren Ende des flexiblen Schafts ein Handgriff vorgesehen, durch den das medizinische Greifgerät gehalten und betätigt werden kann.

Bei diesem medizinischen Greifgerät ist jede einzelne Branche mittels eines eigenen Steuermechanismus gegenüber dem Steg bewegbar. Das heißt, das Greifgerät besitzt mindestens zwei Mäuler, die unabhängig voneinander geöffnet und geschlossen werden können. Somit können mindestens zwei Gewebeabschnitte separat gegriffen und fixiert werden. Der Schaft des Greifgeräts ist flexibel, sodass er möglichst geringe Rückstellkräfte erzeugt, wenn er aus seiner Ausgangsform heraus verformt wird. An dem vorderen Ende des Schafts kann auch ein kleiner Vorsprung vorgesehen sein, an dem der Steg und die Branchen befestigt sein können. Die Branchen können zum Beispiel Widerhaken oder Zähne aufweisen, sie können profiliert oder flach, gerade oder bogenförmig sein. Die Stegelemente können entsprechend so ausgebildet sein, dass sie im Zusammenspiel mit den Branchen ein Herausrutschen des gegriffenen Gewebes aus dem jeweiligen Maul verhindern. Die Branchen und Stegelemente können auch insbesondere so ausgebildet sein, dass sie das gegriffene Gewebe nicht verletzen. Am hinteren Ende des flexiblen Schafts ist ein Handgriff vorgesehen. Dieser Handgriff ermöglicht es, das medizinische Greifgerät präzise zu halten und zu führen und gleichzeitig die Branchen präzise zu betätigen. Der Handgriff kann entweder direkt an dem hinteren Ende des flexiblen Schafts angebracht sein, oder es kann ein Zwischenstück zwischen diesen vorgesehen sein, wie dies im Folgenden beschrieben ist.

Gemäß der Erfindung ist ein Verbindungselement zwischen dem hinteren Ende des flexiblen Schafts und dem vorderen Ende des Handgriffs vorgesehen, wobei das Verbindungselement zu dem flexiblen Schaft hin einen Durchgangskanal aufweist, in dem alle Übertragungselemente der Steuervorrichtungen gemeinsam geführt sind. Der Durchgangskanal in dem Verbindungselement zweigt sich dann so auf, dass zu dem Handgriff hin je ein Durchgangskanal für jedes Übertragungselement vorgesehen ist.

Solch ein Vermindungselement hat den Vorteil, dass die Übertragungselemente, die als zug- und schubsteife Bauteile ausgebildet sind, sich individuell betätigen lassen, ohne dass sie sich gegenseitig so stark beeinflussen, dass eine individuelle und präzise Steuerung beeinträchtigt oder verhindert wird. Genauer gesagt kann es sein, dass dann, wenn kein Verbindungselement vorgesehen ist und die Übertragungselemente direkt in das eine Durchgangsloch des flexiblen Schafts eingeführt sind, eine große Reibung zwischen den Übertragungselementen vorherrscht und somit eine Betätigung des einen Übertragungselements eine Bewegung des anderen verursacht. Die Gefahr einer gegenseitigen Beeinflussung ist gerade im Bereich der Zusammenführung bzw. Einführung der Übertragungselemente besonders groß, da die Übertragungselemente konstruktionsbedingt leicht gebogen sind und gegeneinander gedrückt werden können. Zudem ist eine Schmierung in diesem Bereich gegenüber dem flexiblen Schaft erschwert. Solch ein Verbindungselement kann auch die Reibung der Übertragungselemente gegenüber der umgebenden Wand verringern, indem Material und Geometrie der umgebenden Wand angepasst werden.

Gemäß einer Weiterbildung des medizinischen Greifgeräts ist an der Aufzweigung des Durchgangskanals zu den Durchgangskanälen eine Kammer vorgesehen ist, um die Aufzweigung bzw. Zusammenführung der Übertragungselemente zu vereinfachen.

Solch eine Kammer ist dort angeordnet, wo die Übertragungselemente innerhalb des Verbindungselements die größte Biegung bzw. Krümmung aufweisen. Die Übertragungselemente werden an dem hinteren Ende des Verbindungselements in Durchgangskanäle eingeführt, die sich im Wesentlichen geradlinig in Längsrichtung des Verbindungselements erstrecken. Nichts desto trotz verlaufen diese Durchgangskanäle in radialer Richtung zu dem vorderen Ende hin zusammen und vereinigen sich letztendlich zu einem einzigen Durchgangskanal, der entlang der Mittelachse des Verbindungselements verläuft. Daher weist jeder einzelne Durchgangskanal von dem hinteren Ende zu dem vorderen Ende des Verbindungselements einen gewissen Knick auf. An dieser Stelle werden zudem die Übertragungselemente zusammengeführt. Aufgrund des zuvor beschriebenen Verhaltens der Übertragungselemente bei einer Betätigung der Branchen ist es in diesem Bereich besonders wahrscheinlich, dass sich die Übertragungselemente gegenseitig beeinflussen. Die Kammer ermöglicht nun eine gewisse Ausweichbewegung der Übertragungselemente im Zusammenführungsbereich und verbessert damit die Präzision bei der Betätigung der Branchen.

Gemäß einer Weiterbildung des medizinischen Greifgeräts liegen die Öffnungen der Durchgangskanäle zu dem Handgriff hin in einer Ebene senkrecht zu der Achse des Verbindungselements auf einer Kreisbahn.

Dadurch sind alle Durchgangskanäle im Bereich des hinteren Endes des Verbindungselements im selben Winkel gegenüber dem Durchgangskanal an dem vorderen Ende geneigt, so dass sich die Übertragungselemente bezüglich ihrer Reibung in dem Durchgangskanal nicht unterscheiden.

Gemäß einer Weiterbildung des medizinischen Greifgeräts sind die Öffnungen der Durchgangskanäle zu dem Handgriff hin auf der Kreisbahn gleichmäßig verteilt.

Auf diese Weise sind die Durchgangskanäle rotationssymmetrisch um die Achse des Verbindungselements und somit des flexiblen Schafts angeordnet. Nachdem die Position des Durchgangskanals mit der Position des zugehörigen Betätigungselements korrespondiert, ermöglicht diese Weiterbildung die optimale Verteilung einer Vielzahl von Betätigungselementen bzw. Steuervorrichtungen um die Achse des Greifgeräts herum.

Gemäß einer Weiterbildung des medizinischen Greifgeräts sind die Öffnungen der Durchgangskanäle zu dem Handgriff hin aufgeweitet.

Eine solche Aufweitung der Durchgangskanäle ermöglicht ein sanfteres Einführen der Übertragungselemente in die Durchgangslöcher und verhindert, dass in dem Bereich der Öffnungskante ein Übertragungselement fest hängt. Dies ist insbesondere dann interessant, wenn das Übertragungselement als schub- und zugfeste aber biegeweiche Spirale ausgebildet ist. In diesem Fall könnte sich eine scharfe Öffnungskante des Durchgangslochs zwischen zwei benachbarten Wicklungen der Spirale verhaken. Auf diese Weise lassen sich auch geringe Positionsungenauigkeiten zwischen Durchgangsloch und Betätigungselement ausgleichen.

Gemäß einer Weiterbildung des medizinischen Greifgeräts weist der Handgriff zwei Schienen auf, die sich im Wesentlichen parallel zur Achse des flexiblen Schafts von dessen hinteren Ende aus erstrecken. An jeder Schiene ist ein Schlitten vorgesehen, der entlang der zugehörigen Schiene bewegbar ist. Jeder Schlitten ist mit dem zugehörigen Steuermechanismus schub- und zugfest verbunden, so dass eine Bewegung eines jeden Schlittens entlang der jeweiligen Schiene durch ein zugehöriges Übertragungselement der Steuermechanismen an die zugehörige Branche übertragen wird.

Dies stellt eine besonders vorteilhafte Ausführungsform von Betätigungselementen dar. Der Nutzer kann mit seiner Hand die Schlitten gleichzeitig greifen und somit eine präzise gleichzeitige Steuerung aller Branchen bewirken. Zudem ist die Verwendung von auf Schienen laufenden Schlitten für den Nutzer sehr intuitiv. Die Branchen können statt durch eine Schiebebewegung entlang der Achse des Greifgeräts auch durch Drehbewegungen von z. B. Knöpfen oder am Griff angebrachten Ringen gesteuert werden. Hierbei kann der Nutzer leichter die Öffnungs- bzw. Schließrichtung der Branchen verwechseln. Bei der hier vorgeschlagenen Ausbildung hingegen sind solche Verwechslungen von vornherein so gut wie ausgeschlossen.

Gemäß einer Weiterbildung des medizinischen Greifgeräts ist an jedem Schlitten ein Griffelement vorgesehen.

Um es dem Nutzer einfacher zu machen, das Greifgerät gleichzeitig zu halten, zu führen und präzise zu steuern, sind Griffelemente ab den Schlitten vorgesehen, die sich besonders leicht greifen lassen.

Gemäß einer Weiterbildung des medizinischen Greifgeräts sind die Schienen an ihrem körperfernen Ende miteinander verbunden und ein weiteres Griffelement ist an diesem Verbindungsabschnitt vorgesehen.

Auf diese Weise ist es für den Nutzer noch einfacher, das Greifgerät sicher zu führen.

Gemäß einer Weiterbildung des medizinischen Greifgeräts ist das weitere Griffelement gegenüber der Mittelachse der Schienen versetzt und/oder geneigt, um eine ergonomische Handhaltung eines Greifenden zu ermöglichen.

Die Anordnung der Griffelemente ist nicht von vornherein festgelegt sondern kann an die Ergonomie des Nutzers angepasst sein. Hierbei ist zum Beispiel zu berücksichtigen, ob sich der Nutzer des Greifgeräts eher hinter oder eher über dem Gerät befindet. Dementsprechend ändert sich eine angenehme Handhaltung an dem Handgriff des Greifgeräts.

Gemäß einer Weiterbildung des medizinischen Greifgeräts sind die Griffelemente durch Ösen gebildet, deren Durchgangslöcher zueinander parallele Mittelachsen aufweisen.

Solche Ösen sind eine besonders vorteilhafte Ausführungsform für die Griffelemente. Der Nutzer kann leicht einen Finger durch jede Öse stecken, wodurch er einen sicheren Griff und eine gute taktile Rückmeldung vom Greifgerät erlangt. Ösen eigen sich insbesondere auch als Griffelemente, da ein Abrutschen der Hand bzw. des Fingers effektiv verhindert werden kann. Diese Ösen müssen nicht zwangsläufig kreisförmig sein. Um einen möglichst flachen Aufbau des Handgriffs zu ermöglichen, sind die Ösen bei dieser Ausführungsform so angeordnet, dass ihre Mittelachsen alle zueinander parallel sind. Um eine ergonomische Handhaltung zu ermöglichen ist es auch möglich, eine oder mehrere Ösen zu drehen. Allerdings vergrößert dies die Gesamtdicke des Handgriffs. Eine platzsparende Alternative dazu ist es, die Ösenform z. B. leicht oval zu gestalten und dafür die Mittelachsen der Ösen parallel zu lassen.

Gemäß einer Weiterbildung des medizinischen Greifgeräts umschließt jeder Schlitten die zugehörige Schiene, so dass ein Loslösen des Schlittens von der Schiene verhindert ist.

Dies verhindert sicher, dass sich ein Schlitten von der zugehörigen Schiene löst. Es ist aber auch möglich, den Schlitten mit Hilfe einer Schnapp- oder Klickverbindung auf der Schiene zu befestigen, so dass kein Werkzeug und/oder Montagematerial zum Anbringen des Schlittens erforderlich ist.

Gemäß einer Weiterbildung des medizinischen Greifgeräts ist das Übertragungselement eines jeden Steuermechanismus zwischen den beiden Schienen mit dem jeweiligen Schlitten verbunden.

Auf diese Weise sind die Übertragungselemente im Bereich des Handgriffs möglichst nahe an der Mittelachse des Greifgeräts angeordnet, so dass die Biegung der Durchgangskanäle in dem Verbindungselement möglicht gering ausfallen kann.

Gemäß einer Weiterbildung des medizinischen Greifgeräts sind die Schienen so geformt, dass ein Drehen des zugehörigen Schlittens um die jeweilige Schiene verhindert ist.

Das heißt, die Schienen haben einen nicht kreisförmigen Querschnitt. Dadurch wird zuverlässig verhindert, dass die Griffelemente bei einem Greifen durch den Nutzer ausweichen und eine Benutzung des Greifgerätes somit erschwert wird. Zudem sind Schlitten und Schiene in Umfangsrichtung der Schiene formschlüssig, wobei natürlich ein gewisses Spiel zwischen ihnen vorgesehen ist, um ein Verschieben des Schlittens entlang der Schiene zu gewährleisten.

Gemäß einer Weiterbildung des medizinischen Greifgeräts ist jeder Schlitten auf der zugehörigen Schiene fixierbar, so dass die Betätigungsposition der zugehörigen Branche fixiert ist.

Hierbei kann die Fixierung durch geeignete Auswahl des Reibungskoeffizienten zwischen Schiene und Schlitten erzielt werden, beispielweise durch geeignete Materialauswahl oder Oberflächengestaltung. Alternativ dazu kann aber auch eine Art Feststellbremse als Fixiereinrichtung vorgesehen werden. Diese Alternative hätte den Vorteil, dass die Reibung der gesamten Steuereinrichtung möglichst niedrig gehalten werden kann, so dass der Nutzer eine besonders gute Rückmeldung von den Branchen erhält.

Gemäß einer Weiterbildung des medizinischen Greifgeräts sind die Übertragungselemente mittels Hinterschneidungen an den Schlitten lösbar befestigt.

Dies ist eine Ausführungsform, die eine Verwendung von zusätzlichen Montagemitteln erübrigt. Somit können die Übertragungselemente ohne Werkzeug schnell und sicher an den Schlitten befestigt werden.

Insgesamt kann der Handgriff so aufgebaut sein, dass er durch einfache Steck- und Klickverbindungen zusammengebaut werden kann, ohne dass dafür Werkzeug benötigt wird. Vorzugsweise sind die Einzelteile durch z. B. Spritzguss aus Kunststoff hergestellt.

Gemäß einer Weiterbildung des medizinischen Greifgeräts ist der Steg an dem vorderen Ende des Schafts angelenkt. Dadurch kann der Steg gegenüber dem Schaft geschwenkt werden.

Dies ist vorteilhaft, wenn Gewebe an einer Stelle gegriffen werden soll, an der sehr wenig Platz ist, oder wenn die Normale auf die Ebene, die durch die zu greifenden Geweberänder gebildet wird, nicht mit der Längsachse des Schafts übereinstimmt. Das heißt, die Mäuler können in ihrer Gesamtheit gegenüber dem Schaft geneigt werden, so dass eine noch unbeschränktere Steuerung des medizinischen Greifgeräts gewährleistet ist.

Gemäß einer Weiterbildung des medizinischen Greifgeräts ist ein eigener Steuermechanismus zum Schwenken des Stegs gegenüber dem Schaft vorgesehen.

Dieser Steuermechanismus kann einer der hier aufgezeigten Steuermechanismen sein, wobei für ein Schwenken des Stegs ein zusätzliches Betätigungselement an dem Handgriff vorgesehen sein kann. Das Schwenken des Stegs gegenüber dem Schaft kann in einer oder mehreren Richtungen möglich sein. Für ein Schwenken in mehrere Richtungen sind eventuell mehrere Steuermechanismen erforderlich.

Alle hier aufgezeigten Merkmale und Eigenschaften können beliebig kombiniert werden. Insbesondere können verschiedene Ausführungsformen der Branchen (mit/ohne Schnittkanten, gerade oder gebogen, ...), Stege (ein Stegelement, geteilter Steg, Feder/elastisches Element zwischen Stegelementen, ...) und Steuermechanismen (elektrischer/mechanischer Steuermechanismus, selbsttätig öffnend/schließend,...) mit verschiedenen Handgriffen und/oder Übertragungselementen (elektrische Leitungen, Bowdenzüge, Kombination daraus) kombiniert werden.

Im Folgenden ist der Aufbau der eigentlichen Greifvorrichtung im Detail beschrieben.

Der Steg des medizinischen Greifgeräts besteht aus mindestens zwei Stegelementen, wobei maximal eines der Stegelemente starr an dem Schaft befestigt ist und die restlichen Stegelemente drehbar an dem vorderen Ende des Schafts oder dem maximal einen starren Stegelement angelenkt sind. Das heißt, die Stegelemente sind entweder alle drehbar an dem vorderen Ende des Schafts angelenkt oder ein Stegelement ist starr mit dem vorderen Ende des Schafts verbunden und die restlichen Stegelemente sind drehbar an dem vorderen Ende des Schafts oder an dem einen starren Stegelement angelenkt. Zwischen oder an den Stegelementen ist mindestens ein Element so angeordnet, dass die beweglichen Stegelemente mit Hilfe des Elements zumindest in eine Richtung bewegt werden können. Darüber hinaus ist jede Branche des Greifgeräts an einem Stegelement drehbar angelenkt.

Des Weiteren kann mindestens ein elastisches Element so an den Stegelementen angeordnet sein, dass es die Stegelemente an ihrem körperfernen Ende auseinander drängt. Es kann entweder ein elastisches Element vorgesehen sein, das alle Stegelemente auseinander drängt, oder es können mehrere elastische Elemente vorgesehen sein, die jeweils zwei Stegelemente auseinander drängen. Das mindestens eine elastische Element kann beispielsweise in dem jeweiligen Maul zwischen den Stegelementen angeordnet sein und die Stegelemente auseinander drücken. Dies ist beispielsweise durch Druckfedern oder Gummielemente möglich. Im Gegensatz dazu kann das mindestens eine elastische Element aber auch außerhalb der Mäuler angeordnet sein und die Stegelemente auseinander ziehen. Dies kann zum Beispiel durch eine bogenförmige Zugfeder realisiert werden. Anstelle eines elastischen Elements kann aber auch ein magnetisches Element diese Aufgabe übernehmen, wobei das magnetische Element in den Stegelementen enthalten sein kann. Des Weiteren könnte auch ein anderes Element wie beispielsweise eine Art mikroskopische Luftdruckfeder verwendet werden. Diese Elemente können alternativ auch zwischen den Stegelementen und dem Schaft des Greifgeräts angeordnet sein.

Alternativ zu dem vorstehend beschriebenen Aufbau können aber in Zukunft beispielsweise elektrische Mikromotoren den Abstand zwischen den Stegelementen regulieren. Diese Ausführungsform kann aufgrund der schnellen Entwicklung auf diesem Gebiet bereits bald Anwendung finden.

Die Stegelemente können aber auch gänzlich anders ausgebildet sein. Zum Beispiel können die einzelnen beweglichen Stegelemente aus einem elastischen Material wie beispielsweise Federstahl ausgebildet sein. Diese Stegelemente werden dann an dem Ende, das dem Schaft zugewandt ist, so verbunden, dass sich die zu diesen verbundenen Enden entgegen gesetzten Enden der Stegelemente durch die ihnen eigene Elastizität voneinander entfernen.

Diese Ausbildung ist insbesondere dann sinnvoll, wenn das erfindungsgemäße medizinische Greifgerät im Zusammenspiel mit einem Endoskop verwendet wird. In diesem Fall wird das erfindungsgemäße Greifgerät durch einen Arbeitskanal des Endoskops eingeführt. Die Stegelemente, deren vordere Enden auf eine der zuvor beschriebenen Weisen das Bestreben haben, sich voneinander zu entfernen, können dies uneingeschränkt tun, so lange der gesamte Kopf des erfindungsgemäßen Greifgeräts, der aus den Branchen und den Stegelementen besteht, aus dem Arbeitskanal des Endoskops heraus ragt. Wenn aber der Kopf des Greifgeräts nun ein Stück weit in eine Hülse hinein gezogen wird, die sich an dem Ende des Endoskops befindet, liegen die Stegelemente an der Hülse an und werden bei einem weiteren Hineinziehen des Greifgeräts in die Hülse zu der Achse des Greifgeräts hin verformt. Es kann auch die Hülse bewegt bzw. aufgeschoben werden. Einzig wichtig hierbei ist die relative Bewegung von Greifgerät zu Hülse. Wenn der Kopf des Greifgeräts wieder vollständig aus der Hülse heraus geschoben wird, entfernen sich die vorderen Enden der Stegelemente wieder voneinander. Auf diese Weise kann der Abstand der vorderen Enden der Stegelemente und somit der inneren Ränder der Mäuler voneinander eingestellt werden. Somit kann der Abstand der Stegelemente dem Abstand der zu greifenden Gewebeteile angepasst werden. Die Hülse kann auch ein Teil eines Endoskops sein.

Bei dem medizinischen Greifgerät besitzen die Steuermechanismen jeweils ein zug- und druckfestes Übertragungselement. Die mindestens zwei Übertragungselemente verlaufen gemeinsam in dem Schaft, wobei jedes dieser Übertragungselemente an dem vorderen Ende des Schafts mit einer Branche verbunden ist. Zudem besitzen die Steuermechanismen jeweils eine Steuervorrichtung, die an dem hinteren Ende des Schafts vorgesehen ist und mit dem zugehörigen Übertragungselement verbunden ist. Jedes Übertragungselement überträgt die von der entsprechenden Steuervorrichtung ausgegebene Bewegung an die entsprechende Branche. Die Übertragung der Bewegung schließt auch eine Übertragung von Bewegungssignalen mit ein, die dann zum Beispiel im Kopf des Greifgeräts in Bewegungen umgesetzt werden. Die Signale können elektrische Signale sein, die durch elektrische Leitungen übertragen werden. Als weitere Alternative dazu können die Branchen auch mittels Magneten, elastischen Elementen oder desgleichen selbsttätig geöffnet werden und anstelle des zug- und druckfesten Übertragungselements ist ein nur zugfestes Übertragungselement vorgesehen. Dann findet der Öffnungsvorgang der Mäuler selbsttätig statt und der Schließvorgang wird durch die Übertragungselemente vorgenommen. Der Vorteil ist, dass Übertragungselemente, die nicht druckfest sein müssen, eine geringere Querschnittsfläche erfordern. In diesem Fall müsste allerdings sichergestellt werden, dass sich die Mäuler nicht versehentlich wieder öffnen, wenn zum Beispiel das Griffelement des entsprechenden Übertragungselements versehentlich freigegeben wird. Eine detaillierte Beschreibung möglicher Griffelemente und seiner Alternativen erfolgt im Folgenden.

Die Branchen und/oder die zugehörigen Stegelemente des medizinischen Greifgeräts können auch unterscheidbar sein. Außerdem können die zugehörigen Steuervorrichtungen unterscheidbar sein, wobei eine eindeutige Zuordnung der entsprechenden Branche zu der zugehörigen Steuervorrichtung möglich ist. Auf diese Weise besteht vor einer Betätigung einer Steuervorrichtung Gewissheit darüber, welche Branche betätigt wird. Zudem kann auf diese Weise sicher entschieden werden, welche Steuervorrichtung betätigt werden soll, um ein bestimmtes Maul zu öffnen. Eine Betätigung der falschen Branche kann dazu führen, dass ein bereits gegriffenes Gewebestück verloren geht und dadurch die Gesamtzeit der Operation erheblich verlängert wird. Es kann auch beispielsweise zur Folge haben, dass die Randabschnitte durch das häufige manipulieren ausfransen, sodass der Verschluss der Öffnung nicht mehr sicher durchgeführt werden kann. In diesem Fall muss oft ein offner Zugriff mit all seinen nachteiligen Folgen vorgenommen werden.

Insbesondere sind die Branchen und/oder Stegelemente durch ihre Farbe, ihre Form und/oder ihr Material unterscheidbar. So können zum Beispiel Symbole oder Nuten an den Branchen und/oder den Stegelementen ausgebildet sein, die sich entsprechend an den zugehörigen Steuervorrichtungen wieder finden. Es können auch Gravuren vorgesehen sein. Farben eignen sich zur Unterscheidung nur dann, wenn die Branchen und/oder Stegelemente mit einer Farbkamera betrachtet werden können. Das Material kann zur Unterscheidung hergezogen werden, wenn es zum Beispiel eine unterschiedliche Oberflächenbeschaffenheit besitzt, wie zum Beispiel eine unterschiedliche Rauheit, eine unterschiedliche Reflexion oder eine unterschiedliche Farbe. Eine Unterschiedliche Durchsichtigkeit des Materials kann auch zur Unterscheidung herangezogen werden. Es ist wahrscheinlich, dass zukünftig vermehrt Kunststoffe für medizinische Zwecke verwendet werden. Diese können unter Umständen transparent sein.

Bei dem medizinischen Greifgerät können die Branchen und/oder die Stegelemente Schnittkanten aufweisen. Mit diesen Schnittkanten können zum Beispiel Gewebeteile durchtrennt werden oder es kann eine Naht aufgetrennt werden.

Vorteilhafter Weise sind die Branchen und die Stegelemente symmetrisch angeordnet und die Branchen sind in radialer Richtung zu der Achse des Greifgeräts drehbar. Das heißt, dass die Branchen und die Stegelemente in Umfangsrichtung des Greifgeräts gleichmäßig verteilt angeordnet sind.

Die Stegelemente können auch in radialer Richtung zu der Achse des Greifgeräts drehbar sein. Alternativ dazu können sich die Branchen in tangentialer Richtung zu der Achse des Greifgeräts oder in einer anderen Richtung zu dieser bewegen.

Üblicherweise sind der Steg und die Branchen zu ihrem vorderen Ende hin gleich lang. Sie können aber auch so ausgebildet sein, dass der Steg zum Beispiel weiter nach vorne vorsteht als die Branchen. Die einzelnen Stegelemente und die einzelnen Branchen können aber auch untereinander unterschiedlich lang ausgebildet sein. Vorteilhafte Wirkungen der Erfindung

### KURZE BESCHREIBUNG DER ABBILDUNGEN DER ZEICHNUNGEN

Diese und weitere Aufgaben, Merkmale und Vorteile der vorliegenden Erfindung sind aus den Ausführungsbeispielen ersichtlich, die im Folgenden unter Bezugnahme auf die beigefügten Figuren detailliert beschrieben sind.
Fig. 1 ist eine seitliche Ansicht eines Kopfs eines medizinischen Greifgeräts gemäß einem ersten Ausführungsbeispiel, dem beide Mäuler geöffnet sind.
Fig. 2 ist eine seitliche Ansicht des Kopfs des medizinischen Greifgeräts gemäß dem ersten Ausführungsbeispiel, bei dem ein Maul geöffnet ist.
Fig. 3A, 3B und 3C sind schematische Darstellungen eines Kopfs eines medizinischen Greifgeräts gemäß einem fünften Ausführungsbeispiel in verschiedenen Zuständen.
Fig. 4A, 4B und 4C sind schematische Darstellungen eines Kopfs eines medizinischen Greifgeräts gemäß einem sechsten Ausführungsbeispiel in verschiedenen Zuständen.
Fig. 5A und 5B sind seitliche Ansichten eines medizinischen Greifgeräts gemäß einem zweiten Ausführungsbeispiel der vorliegenden Erfindung, wobei in der Fig. 5A die Mäuler geöffnet und die Stegelemente beabstandet sind und in der Fig. 5B die Mäuler geschlossen sind und die Stegelemente zusammengedrückt sind.
Fig. 6 ist eine perspektivische Ansicht des Verbindungselements gemäß der Erfindung, bei der ein Teil des Verbindungselements als Gittermodell gezeigt ist.
Fig. 7 ist eine seitliche Ansicht des hinteren Bereichs des Verbindungselements und des vorderen Bereichs des Handgriffs.
Fig. 8 ist eine seitliche Ansicht des Handgriffs.
Fig. 9 ist eine perspektivische Ansicht eines hinteren Endes des Handgriffs mit Griffelement.
Fig. 10 ist eine perspektivische Ansicht des Handgriffs.
Fig. 11 ist eine vergrößerte Ansicht eines Teils der Fig. 10.

### WEGE ZUR AUSFÜHRUNG DER ERFINDUNG

### (erstes Ausführungsbeispiel)

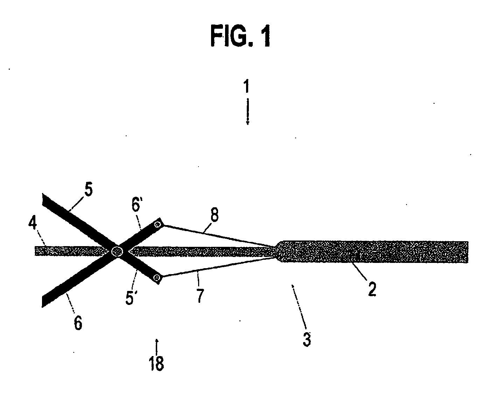

Im Folgenden ist ein erstes Ausführungsbeispiel des medizinischen Greifgeräts der vorliegenden Erfindung unter Bezugnahme auf die Fig. 1, 2 und 6 bis 11 beschrieben.

Das medizinische Greifgerät (1) des ersten Ausführungsbeispiels besitzt einen flexiblen Schaft (2) mit einem vorderen Ende (3) und einem hinteren Ende (14). Der Schaft (2) ist als eine druckfeste biegeweiche Spirale ausgebildet. Zudem besitzt es einen Steg (4) bestehend aus einem einzigen Stegelement, das an dem vorderen Ende (3) des Schafts (2) befestigt ist. Zwei Branchen (5, 6) sind an dem Steg (4) drehbar angelenkt. Teile von zwei flexiblen Steuermechanismen (7, 151, 155; 8, 152, 156) sind in dem Schaft (2) angeordnet und jede einzelne Branche (5, 6) ist mittels eines eigenen Steuermechanismus (7, 151, 155; 8, 152, 156) gegenüber dem Steg (4) bewegbar. In diesem Fall ist die in der Fig. 1 linke Branche (6) durch den Steuermechanismus (8, 152, 156) steuerbar und die in der Fig. 1 rechte Branche (5) ist durch den Steuermechanismus (7, 151, 155) steuerbar. Bei dem ersten Ausführungsbeispiel des medizinischen Greifgeräts besitzen die Steuermechanismen (7, 151, 155; 8, 152, 156) jeweils ein zug- und druckfestes Übertragungselement (7, 8), die gemeinsam in dem Schaft (2) verlaufen. Jedes dieser Übertragungselemente (7, 8) ist an dem vorderen Ende (3) des Schafts (2) mit einer Branche (5, 6) verbunden. Zudem besitzen die Steuermechanismen (7, 151, 155; 8, 152, 156) jeweils eine Steuervorrichtung (151, 155; 152, 156), die an dem hinteren Ende (14) des Verbindungselements (160) vorgesehen und mit dem zugehörigen Übertragungselement (7, 8) verbunden sind, wobei jedes Übertragungselement (7, 8) die von der entsprechenden Steuervorrichtung (151, 155; 152, 156) ausgegebene Bewegung an die entsprechende Branche (5, 6) überträgt.

Bei diesem Ausführungsbeispiel sind die Übertragungselemente (7, 8) Bowdenzüge und die Steuervorrichtungen (151, 155; 152, 156) bestehen aus Schienen (151, 152) und Schlitten (155, 156) mit Griffelementen (155G, 156G). Die Griffelemente (155G, 156G) werden in eine Richtung betätigt, um die entsprechende Branche (5, 6) zu öffnen, und sie werden in die entgegengesetzte Richtung betätigt, um die entsprechende Branche (5, 6) zu schließen. Um die Branchen zu öffnen, werden die Schlitten zu dem Schaft (2) hin bewegt. Die Griffelemente (155G, 156G) sind an einem gemeinsamen Handgriff (150) vorgesehen.

Die Branchen (5, 6) und/oder das zugehörige Stegelement (4) sind bei diesem Ausführungsbeispiel nicht unterscheidbar. Allerdings können bei einem medizinischen Greifgerät (1) gemäß diesem Ausführungsbeispiel auch noch nachträglich Nuten oder Gravuren an den Branchen (5, 6) und/oder dem Stegelement (4) und den zugehörigen Griffelementen (155G, 156G) oder Schlitten (155, 156) vorgesehen werden, so dass dann eine eindeutige Zuordnung von Griffelement (155G, 156G) zu Branche (5, 6) möglich ist. Da bei diesem Ausführungsbeispiel nur ein Stegelement (4) vorgesehen ist, kann die Gravur oder Nut zur Unterscheidung beispielsweise an beiden Seitenflächen des Stegelements (4) vorgesehen sein, wobei zum Beispiel eine Nut an beiden Seitenflächen des Stegelements (4) so vorgesehen wird, dass sie näher zu der Branche (5) ist, und das zu der Branche (5) zugehörige Griffelement (155G) ebenfalls mit einer Nut versehen wird.

Die Branchen (5, 6) und das eine Stegelement (4) sind symmetrisch angeordnet. Das heißt, die Längsachse des eines Stegelements (4) ist mit der Längsachse des medizinischen Greifgeräts (1) identisch und die beiden Branchen (5, 6) sind diametral gegenüberliegend an dem Stegelement (4) angelenkt. Die Branchen (5, 6) sind zudem in radialer Richtung zu der Achse des Greifgeräts (1) drehbar.

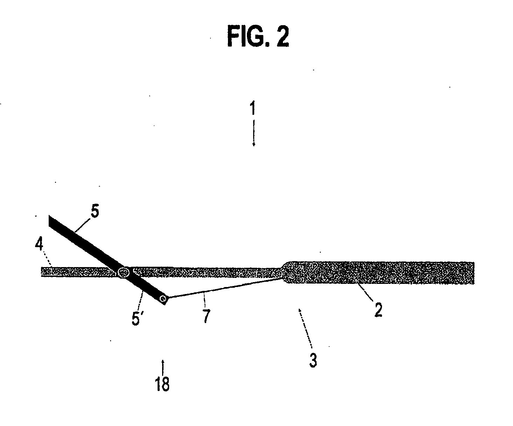

Wie dies in den Fig. 1 und 2 gezeigt ist, sind die Bowdenzüge (7, 8) an Fortsätzen bzw. Hebeln (5', 6') der Branchen (5, 6) befestigt. Diese Fortsätze bzw. Hebel (5', 6') sind so ausgebildet, dass sie sich in einer Aussparung des Stegelements (4) befinden, wenn die Branchen (5, 6) geschlossen sind. Dadurch kann der Durchmesser des Kopfs (18) des Greifgeräts (1) im geschlossenen Zustand sehr klein gehalten werden. Der Kopf (18) des Greifgeräts (1) bezeichnet die Elemente, die an dem vorderen Ende (3) des Greifgeräts (1) vorgesehen sind.

Die Bowdenzüge (7, 8) sind mittels Gelenkmechanismen an den Fortsätzen (5', 6') der Branchen (5, 6) befestigt. Das vordere Ende des Stegelements (4) ist etwas verdickt und die vorderen Enden der Branchen (5, 6) sind leicht zu dem Stegelement (4) hin so gebogen und verjüngt, dass sie in das vordere Ende des Stegelements (4) eingreifen, in dem eine entsprechende Vertiefung vorgesehen ist. Die beiden Branchen (5, 6) sind gleich groß und an derselben Achse an dem Stegelement (4) angelenkt.

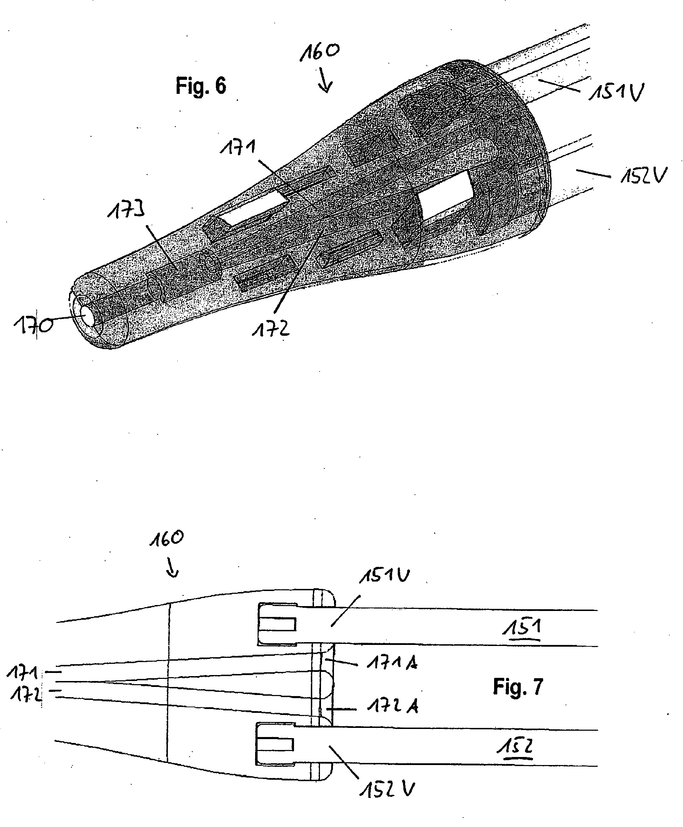

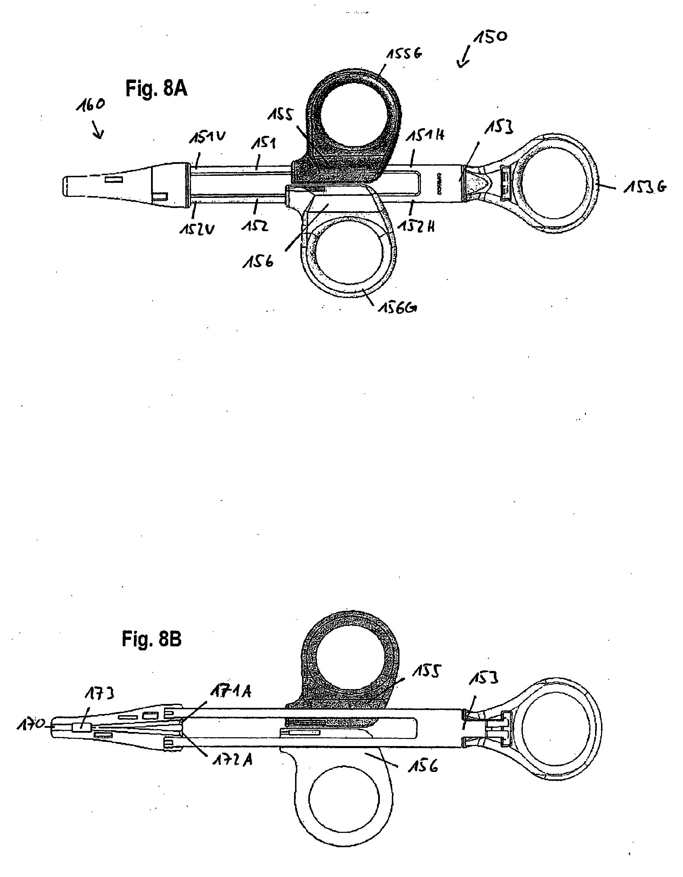

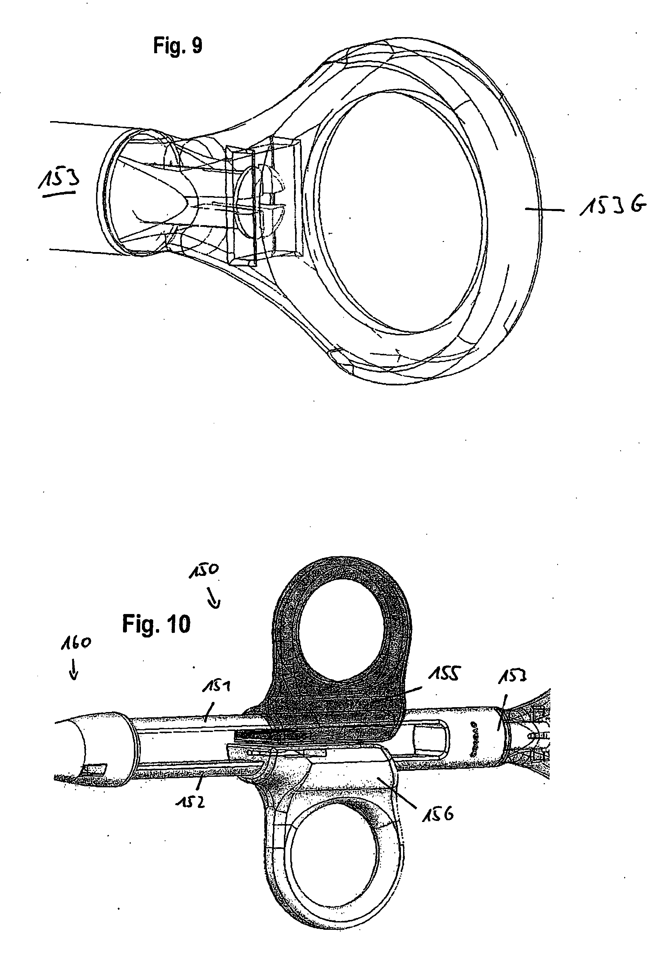

An dem hinteren Ende (14) des Schafts (2) ist bei diesem Ausführungsbeispiel ein Verbindungselement (160) ausgebildet, wie dies in den Fig. 6 und 7 gezeigt ist, an das sich der Handgriff (150) anschließt, der im Wesentlichen aus den Schienen (151, 152), dem Verbindungselement (153), den Schlitten (155, 156) und den Griffelementen (155G, 156G, 153G) besteht. Der Handgriff (150) ist insbesondere in den Fig. 8 und 10 gezeigt.

In dem Verbindungselement sind Durchgangskanäle (170, 171, 172) vorgesehen. Genauer gesagt spaltet sich der Durchgangskanal (170) in einer Kammer (173) in die zwei Durchgangskanäle (171, 172) auf, die V-förmig auseinander laufen und jeweils ein Übertragungselement (7, 8) in sich führen. Das Verbindungselement (160) besteht aus zwei Teilen, die ebenfalls durch eine Steckverbindung verbunden sind. Zu dem Handgriff (150) hin sind die Durchgangslöcher (171, 172) aufgeweitet, wie dies in der Fig. 7 gut zu erkennen ist. Dies ermöglicht ein sanftes Einschieben der Übertragungselemente (7, 8) in das entsprechende Durchgangsloch (171, 172) bei einer Betätigung der jeweiligen Branche (5, 6).

An dem Verbindungselement (160) sind die beiden Schienen (151, 152) mit ihren vorderen Enden (151V, 152V) befestigt, wie dies insbesondere in der Fig. 7 gezeigt ist. Hierbei sind die Schienen (151, 152) mit ihren vorderen enden (151V, 152V) in dem Verbindungselement (160) eingerastet. Auf jede Schiene (151, 152) ist ein Schlitten (155, 156) so aufgesetzt, dass jeder Schlitten (155, 156) die zugehörige Schiene (151, 152) umgreift. Die beiden Schienen sind an ihren hinteren enden (151H, 152H) durch einen Verbindungsabschnitt (153) miteinander verbunden, wobei an diesem Verbindungsabschnitt (153) und an jedem Schlitten (155, 156) ein Griffelement (155G, 156G, 153G) in Form einer im Wesentlichen kreisförmigen Öse vorgesehen ist (das Griffelement 153G ist leicht oval). Bei diesem Ausführungsbeispiel befinden sich die Griffelemente (155G, 156G, 153G) alle in derselben Ebene. Das Griffelement (153G) ist an dem Verbindungsabschnitt (153) ebenfalls mit einer Steckverbindung befestigt.

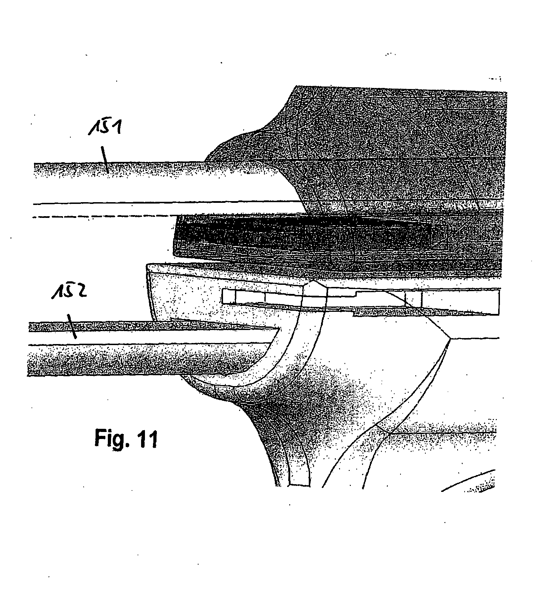

Die Übertragungselemente (7, 8) sind mit den Schlitten (155, 156) im Bereich zwischen den Schienen (151, 152) verbunden. In der Fig. 10 sind Aussparungen gezeigt, in die die Übertragungselemente (7, 8) eingeführt werden und dann mittels Hinterschneidungen befestigt werden. In der Fig. 11 sind die Übertragungselemente (7, 8) selbst nicht gezeigt.

Bei diesem Ausführungsbeispiel ist der gesamte Handgriff aus Kunststoff durch Spritzguss hergestellt. Zudem sind alle Verbindungen durch Steckverbindungen realisiert, wodurch der Zusammenbau des Handgriffs (150) und die Befestigung der Übertragungselemente (7, 8) an den Schlitten (155, 156) ohne Werkzeug bewerkstelligt werden kann. Die Schlitten (155, 156) bestehen aus zwei Halbschalen, die vergleichbar wie das Verbindungselement (160) zusammengeklickt werden können, wobei sie die entsprechende Schiene (151, 152) umgreifen. Die Form der Schienen (151, 152) und der zugehörigen Aussparungen in den Schlitten (155, 156) verhindern, das sich die Griffelemente (155G, 156G) beim Greifen seitlich wegdrehen. Auch die Klickverbindung des hinteren Griffelements (153G) ist so gestaltet, dass sich das Griffelement (153G) nicht verdrehen kann.

Die vordere Öffnung des Durchgangslochs (170) steht zudem in direktem Kontakt mit dem flexiblen Schaft (2).

Dieses Ausführungsbeispiel ist auf ein medizinisches Greifgerät mit zwei Mäulern gerichtet. Ein ähnlicher Aufbau kann aber auch für medizinische Greifgeräte mit drei oder mehr Mäulern verwendet werden. Dazu sind lediglich entsprechend viele Schienen, Schlitten, Durchgangslöcher in dem Verbindungselement und Übertragungselemente vorzusehen, welche vorteilhafter Weise symmetrisch zu der Mittelachse des Greifgeräts anzuordnen sind.

### (zweites Ausführungsbeispiel)

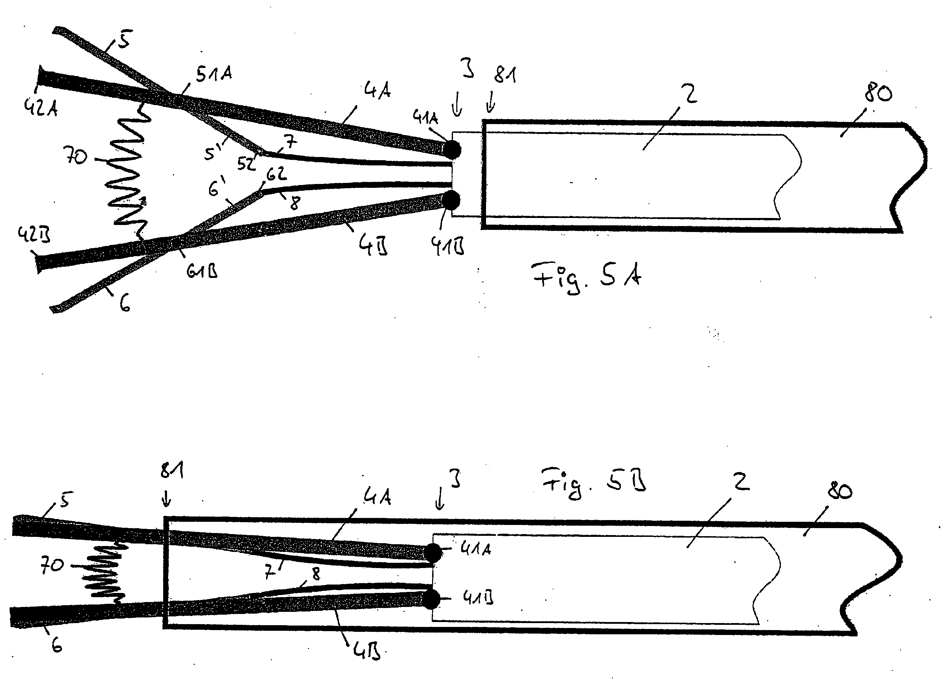

Ein zweites Ausführungsbeispiel des medizinischen Greifgeräts ist unter Bezugnahme auf die Fig. 5A und 5B beschrieben.

Das medizinische Greifgerät (1) des zweiten Ausführungsbeispiels besitzt auch einen flexiblen Schaft (2) mit einem vorderen Ende (3) und einem hinteren Ende (14), einen Steg (4) bestehend aus zwei Stegelementen (4A, 4B), wobei die Stegelemente (4A, 4B) an dem vorderen Ende (3) des Schafts (2) befestigt sind, zwei Branchen (5, 6), die an den Stegelementen (4A, 4B) an den Anlenkpunkten (51A, 61B) angelenkt sind, und zwei flexible Steuermechanismen (7, 151, 155; 8, 152, 156), die zumindest teilweise in dem Schaft (2) angeordnet sind. Die Branche (5) ist durch den Steuermechanismus (7, 151, 155) gegenüber dem Stegelement (4A) drehbar und die Branche (6) ist durch den Steuermechanismus (8, 152, 156) gegenüber dem Stegelement (4B) drehbar. Bei diesem Ausführungsbeispiel besteht der Steg (4) aus zwei Stegelementen (4A, 4B), wobei keines der Stegelemente (4A, 4B) starr an dem Schaft befestigt ist. Ein Element (70) ist an den Stegelementen (4A, 4B) so angeordnet ist, dass die Stegelemente (4A, 4B) relativ zueinander um die Anlenkpunkte (41A, 41B) herum gedreht werden können. Das Element (70) ist ein elastisches Element, das heißt in diesem Fall eine Druckfeder. Die Druckfeder (70) drängt die körperfernen Enden (42A, 42B) der Stegelemente (4A, 4B) auseinander. Wenn allerdings, wie dies in der Fig. 5B gezeigt ist, das Greifgerät (1) ein Stück weit in einen Arbeitskanal (80) eines Endoskops hinein gezogen wird, werden die Stegelemente (4A, 4B) durch die Bewegung und die Anlage an dem Rand (81) des Arbeitskanals (80) aufeinander zu gedrückt und die Druckfeder (70) wird auch zusammen gedrückt. Wie bei dem ersten Ausführungsbeispiel besitzen die Steuermechanismen (7, 151, 155; 8, 152, 156) jeweils ein zug- und druckfestes Übertragungselement (7, 8), die gemeinsam in dem Schaft (2) verlaufen, wobei jedes dieser Übertragungselemente (7, 8) an dem vorderen Ende (3) des Schafts (2) mit einer Branche (5, 6) verbunden ist. Genauer gesagt ist das Übertragungselement (7) an dem Befestigungspunkt (52) an dem Hebel (5') der Branche (5) gelenkig befestigt und das Übertragungselement (8) an dem Befestigungspunkt (62) an dem Hebel (6') der Branche (6) gelenkig befestigt. Jedes Übertragungselement (7, 8) überträgt die von der entsprechenden Steuervorrichtung (7, 151, 155; 8, 152, 156) ausgegebene Bewegung an die entsprechende Branche (5, 6).

Bei dem zweiten Ausführungsbeispiel sind die Übertragungselemente (7, 8) Bowdenzüge. Die Griffelemente (155G, 156G) sind in Form und Funktion bis auf die Tatsache identisch zu den Griffelementen (155G, 156G) des ersten Ausführungsbeispiels, dass das Griffelement (155G) eine Nut aufweist. Auch bei dem zweiten Ausführungsbeispiel sind die Griffelemente (155G, 156G) an einem Griff (150) vorgesehen.

Die Branchen (5, 6) sind unterscheidbar, wobei die Branche (5) an ihrer Außenseite eine ähnliche Nut wie das Griffelement (155G) aufweist. Somit ist für den Nutzer erkennbar, welches Griffelement (155G, 156G) welche Branche (5, 6) steuert.

Wie dies in den Fig. 5A und 5B gezeigt ist, sind die Branchen (5, 6) und die Stegelemente (4A, 4B) symmetrisch angeordnet und die Branchen (5, 6) sind in radialer Richtung zu der Achse des Greifgeräts (1) um die Anlenkpunkte (51A, 61B) herum drehbar. Die Stegelemente (4A, 4B) sind um die Anlenkpunkte (41A, 41B) herum in radialer Richtung zu der Achse des Greifgeräts (1) drehbar.

Mit dem Greifgerät (1) des zweiten Ausführungsbeispiels können dieselben Effekte wie mit dem Greifgerät (1) des ersten Ausführungsbeispiels erzielt werden. Zudem kann durch die Ausbildung des Stegs (4) aus zwei Stegelementen (4A, 4B) der Abstand der beiden Mäuler des Greifgeräts (1) eingestellt werden, wenn das Greifgerät (1) zusammen mit einem Endoskop verwendet wird. Dann kann der Kopf (18) des Greifgeräts (1) des zweiten Ausführungsbeispiels so weit in den Arbeitskanal (80) des Endoskops hinein gezogen werden, dass die Stegelemente (4A, 4B) an dem vorderen Rand (81) des Arbeitskanals (80) des Endoskops anliegen und durch diesen Rand (81) zusammen gedrückt werden. Das Hineinziehen des Kopfs (18) des Greifgeräts (1) wird dadurch erreicht, dass man an dem anderen Ende des Arbeitskanals (80) an dem Schaft (2) des Greifgeräts (1) zieht.

### (drittes Ausführungsbeispiel)

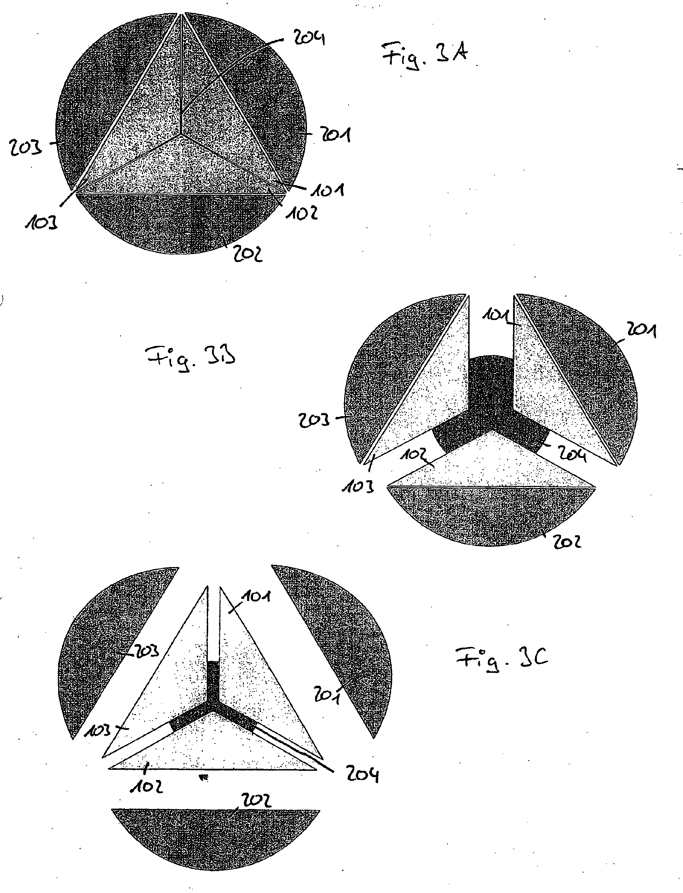

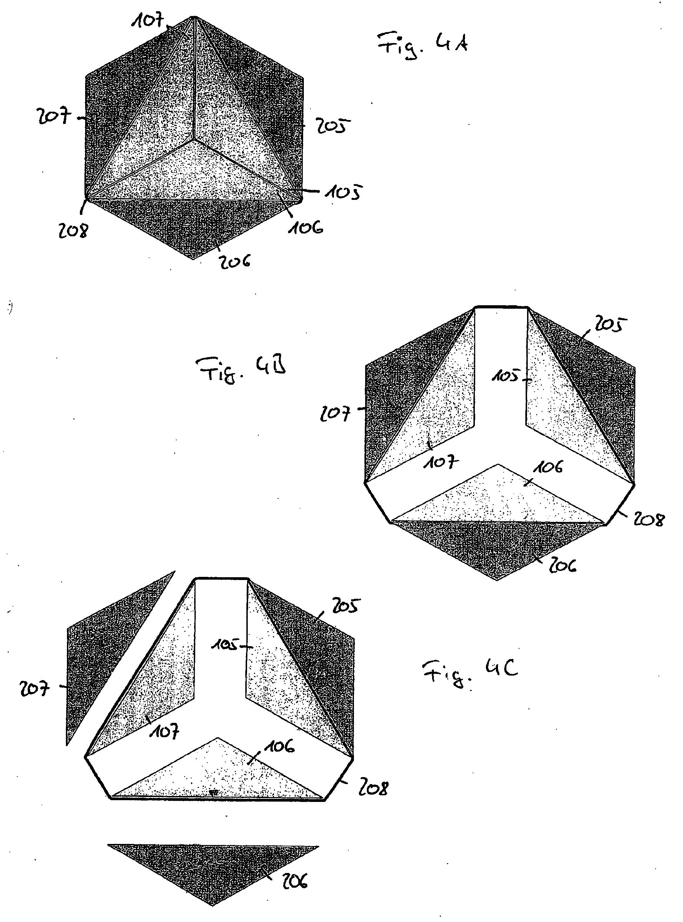

Bei dem dritten Ausführungsbeispiel des Greifgeräts (1), das in den Fig. 4A bis 4C gezeigt ist, unterscheidet sich die Form der Branchen (205, 206, 207) von der der Fig. 3A bis 3C. Zudem werden bei diesem Ausführungsbeispiel die Stegelemente (105, 106, 107) durch ein Gummiband (208) zusammen gezogen. Bei diesem Ausführungsbeispiel sind die Stegelemente (105, 106, 107) so ausgebildet, dass sie elektromagnetische Einrichtungen besitzen, die die Stegelemente (105, 106, 107) auseinander drängen, wenn sie aktiviert sind. Die Stärke der magnetischen Abstoßung lässt sich über den zugeführten Strom einstellen. Das Gummiband (208) besitzt eine vorgegebene Elastizität. Daher kann über die eingestellte magnetische Abstoßung der Stegelemente (105, 106, 107) der Abstand der Stegelemente zueinander eingestellt werden. Die Übertragungselemente für die Branchen (205, 206, 207) dieses Ausführungsbeispiels sind wie bei den vorangehenden Ausführungsbeispielen Bowdenzüge. Die elektrischen Leitungen für die elektromagnetischen Einrichtungen sind bei diesem Ausführungsbeispiel in dem Mantel des Schafts (2) des Greifgeräts (1) untergebracht. Der Griff (150) des Greifgeräts (1) dieses Ausführungsbeispiels verfügt zudem über ein Drehelement, mit dessen Hilfe der elektrische Strom für die elektromagnetischen Einrichtungen eingestellt werden kann.

### (weitere Ausführungsbeispiele)

Gemäß einem weiteren Ausführungsbeispiel können die Branchen (205, 206, 207) des dritten Ausführungsbeispiels auch mittels Miromotoren betätigt werden. Dann kann der Griff (150) zum Beispiel so ausgebildet sein, dass er drei Schlitten, die die Öffnung der Branchen steuern, und ein Drehelement besitzt, das den Abstand der Stegelemente (105, 106, 107) steuert. Die Schlitten sind dann zum Beispiel als lineare Potentiometer ausgebildet.

Gemäß einem weiteren Ausführungsbeispiel kann aber auch der Abstand der Stegelemente zueinander mittels eines Bowdenzugs eingestellt werden.

Im Falle von mechanischen Übertragungselementen können die Schlitten (155, 156) auch mit Federn zu dem Verbindungselement (160) hin vorgespannt sein, so dass dann, wenn die Schlitten freigegeben sind (d.h. momentan keine Betätigung erfolgt und keine Fixiereinrichtung vorhanden ist), die Schlitten durch die Federspannung zu dem Verbindungselement (160) hin bewegt werden, so dass sich die zugehörigen Branchen (5, 6) öffnen. Die Schlitten können auch in der entgegengesetzten Richtung vorgespannt sein, so dass sie sich selbständig schließen können.

Das Greifgerät (1), das in dieser Beschreibung nur für medizinische Zwecke beschrieben ist, kann beispielsweise auch in der Feinmechanik eingesetzt werden.

## Patentansprüche

1. Medizinisches Greifgerät (1) mit einem Schaft (2) mit einem vorderen Ende (13) und einem hinteren Ende (14),
einem Steg (4) bestehend aus mindestens einem Stegelement, wobei der Steg (4) an dem vorderen Ende (3) des Schafts (2) befestigt ist,
mindestens zwei Branchen (5, 6), die an dem Steg (4) angelenkt sind, und mindestens zwei Steuermechanismen (7, 151, 155, 8, 152, 156), die zumindest teilweise in dem Schaft (2) angeordnet und in diesem Abschnitt als Bowdenzüge ausgebildet sind, wobei
jede einzelne Branche (5, 6) mittels eines eigenen Steuermechanismus (7, 151, 155, 8,152,156) gegenüber dem Steg (4) individuell bewegbar ist, und wobei
an dem hinteren Ende (14) des Schafts (2) ein Handgriff (150)
vorgesehen ist, durch den das medizinische Greifgerät (1) gehalten und betätigt werden kann,**dadurch gekennzeichnet, dass** der Schaft (2) flexibel ist, die mindestens zwei Steuermechanismen (7, 151, 155, 8, 152, 156) zumindest teilweise flexibel sind, und
ein Verbindungselement (160) zwischen dem hinteren Ende (14) des flexiblen Schafts (2) und dem vorderen Ende (151V, 152V) des Handgriffs (150) vorgesehen ist, wobei
das Verbindungselement (160) zu dem flexiblen Schaft (2) hin einen Durchgangskanal (170) aufweist, in dem alle Übertragungselemente (7, 8) der Steuervorrichtungen (7, 151, 155, 8, 152, 156) geführt sind, und sich der Durchgangskanal (170) in dem Verbindungselement (160) so aufzweigt, dass zu dem Handgriff (150) hin je ein Durchgangskanal (171, 172) für jedes Übertragungselement (7, 8) vorgesehen ist.

2. Medizinisches Greifgerät (1) gemäß Anspruch 1,
**dadurch gekennzeichnet, dass**
an der Aufzweigung des Durchgangskanals (170) zu den Durchgangskanälen (171, 172) eine Kammer (172) vorgesehen ist, um die Aufzweigung bzw. Zusammenführung der Übertragungselemente (7, 8) zu vereinfachen.

3. Medizinisches Greifgerät (1) gemäß Anspruch 1 oder 2,
**dadurch gekennzeichnet, dass**
die Öffnungen der Durchgangskanäle (171, 172) zu dem Handgriff (150) hin in einer Ebene senkrecht zur Achse des Verbindungselements (160) auf einer Kreisbahn liegen.

4. Medizinisches Greifgerät (1) gemäß Anspruch 2,
**dadurch gekennzeichnet, dass**
die Öffnungen der Durchgangskanäle (171, 172) zu dem Handgriff (150) hin auf der Kreisbahn gleichmäßig verteilt sind.

5. Medizinisches Greifgerät (1) gemäß Anspruch 3 oder 4,
**dadurch gekennzeichnet, dass**
die Öffnungen (171A, 172A) der Durchgangskanäle (171, 172) zu dem Handgriff (150) hin aufgeweitet sind.

## Claims

1. A medical gripping device (1) comprising a shaft (2) having a front end (13) and a rear end (14),
a web (4) consisting of at least one web element, the web (4) being mounted at the front end (3) of the shaft (2),
at least two branches (5, 6) hinged at the web (4), and
at least two control mechanisms (7, 151, 155, 8, 152, 156) arranged at least partially in the shaft (2) and being configured as Bowden cables in this portion, wherein
each single branch (5, 6) is individually movable vis-à-vis the web (4) by means of a separate control mechanism (7, 151, 155, 8, 152, 156), and wherein
at the rear end (14) of the shaft (2) a handle (150) is provided by which the medical gripping device (1) can be held and actuated, **characterized in that**
the shaft (2) is flexible, the at least two control mechanisms (7, 151, 155, 8, 152, 156) are at least partially flexible, and
a connecting element (160) is provided between the rear end (14) of the flexible shaft (2) and the front end (151V, 152V) of the handle (150), wherein
the connecting element (160) has a through passage (170) toward the flexible shaft (2) in which through passage (170) all transmission elements (7, 8) of the control devices (7, 151, 155, 8, 152, 156) are guided and the through passage (170) is branched in the connecting element (160) so that a respective through passage (171, 172) for each transmission element (7, 8) is provided toward the handle (150).

2. The medical gripping device (1) according to claim 1,
**characterized in that**
at the branching of the through passage (170) to the through passages (171, 172) a chamber (172) is provided for facilitating the branching or combining of the transmission elements (7, 8).

3. The medical gripping device (1) according to claim 1 or 2,
**characterized in that**
the openings of the through passages (171, 172) toward the handle (150) are located in a plane perpendicular to the axis of the connecting element (160) on an orbit.

4. The medical gripping device (1) according to claim 2,
**characterized in that**
the openings of the through passages (171, 172) toward the handle (150) are evenly spaced on the orbit.

5. The medical gripping device (1) according to claim 3 or 4,
**characterized in that**
the openings (171A, 172A) of the through passages (171, 172) are widened toward the handle (150).

## Revendications

1. Appareil de préhension (1) médical comprenant une tige (2) dotée d'une extrémité avant (13) et d'une extrémité arrière (14),
une entretoise (4) constituée d'au moins un élément d'entretoise, sachant que l'entretoise (4) est fixée au niveau de l'extrémité avant (3) de la tige (2),
au moins deux branches (5, 6), qui sont articulées au niveau de l'entretoise (4), et
au moins deux mécanismes de commande (7, 151, 155, 8, 152, 156), qui sont disposés au moins en partie dans la tige (2) et qui sont réalisés dans ladite section sous la forme de câbles Bowden,
sachant que chaque branche (5, 6) peut être déplacée individuellement par rapport à l'entretoise (4) au moyen d'un mécanisme de commande (7, 151, 155, 8, 152, 156) propre, et
sachant qu'une poignée (150) est prévue au niveau de l'extrémité arrière (14) de la tige (2), par laquelle l'appareil de préhension (1) médical est tenu et peut être activé, **caractérisé en ce que** la tige (2) est flexible, **en ce que** les au moins deux mécanismes de commande (7, 151, 155, 8, 152, 156) sont au moins en partie flexibles, et
**en ce qu'**un élément de liaison (160) est prévu entre l'extrémité arrière (14) de la tige flexible (2) et l'extrémité avant (151V, 152V) de la poignée (150),
sachant que l'élément de liaison (160) présente, en direction de la tige (2) flexible, un canal de passage (170), dans lequel tous les éléments de transmission (7, 8) des dispositifs de commande (7, 151, 155, 8, 152, 156) sont guidés, et sachant que le canal de passage (170) bifurque dans l'élément de liaison (160) de telle manière que respectivement un canal de passage (171, 172) est prévu pour chaque élément de transmission (7, 8) en direction de la poignée (150).

2. Appareil de préhension (1) médical selon la revendication 1,
**caractérisé en ce**
**qu'**est prévue au niveau de la bifurcation du canal de passage (170) en direction des canaux de passage (171, 172) une chambre (172) afin de simplifier la bifurcation ou l'assemblage des éléments de transmission (7, 8).

3. Appareil de préhension (1) médical selon la revendication 1 ou 2,
**caractérisé en ce**
**que** les orifices des canaux de passage (171, 172) se trouvent en direction de la poignée (150) dans un plan de manière perpendiculaire par rapport à l'axe de l'élément de liaison (160) sur une trajectoire circulaire.

4. Appareil de préhension (1) médical selon la revendication 2,
**caractérisé en ce**
**que** les orifices des canaux de passage (171, 172) sont répartis de manière homogène sur la trajectoire circulaire en direction de la poignée (150).

5. Appareil de préhension (1) médical selon la revendication 3 ou 4,
**caractérisé en ce**
**que** les orifices (171A, 172A) des canaux de passage (171, 172) s'élargissent en direction de la poignée (150).
